# EUROPEAN PATENT SPECIFICATION

(11) **EP 2 144 616 B1**
(45) Date of publication and mention of the grant of the patent: **18.08.2010**
(21) Application number: 08736020.2
(22) Date of filing: 09.04.2008
(51) Int. Cl.: A61K 33/00, A61P 25/00

(54) **USE OF HYPERBARIC CONDITIONS TO PROVIDE NEUROPROTECTION**
VERWENDUNG HYPERBARISCHER BEDINGUNGEN ZUR BEREITSTELLUNG VON NEUROPROTEKTION
UTILISATION DE CONDITIONS HYPERBARES POUR ASSURER UNE NEUROPROTECTION

(30) Priority: 10.04.2007 EP 07251539
(43) Date of publication of application: 20.01.2010
(73) Proprietor: Air Products and Chemicals, Inc., Allentown, PA 18195-1501 (US)
(72) Inventor: FRANKS, Nicholas, Peter, London SW7 2BZ (GB); MAZE, Mervyn, London SW10 9NH (GB); SACRISTAN MARTIN, Juan Carlos, 28700 Madrid (ES)
(74) Representative: Muir, Benjamin M. J.
(86) International application number: PCT/EP2008/054296
(87) International publication number: WO 2008/122654

(56) References cited:
- EP-A1- 0 551 898
- US-A- 4 622 976
- US-A1- 2005 152 988
- US-B1- 6 559 190
- WINKLER: "Xenon as an inhalation contrast medium under hyperbaric conditions." THE AMER.J.ROENTGENOLOGY, vol. 104, no. 4, 1968, - 1968 pages 756-761, XP002438998

## Description

The present invention relates to the use of hyperbaric conditions, and in particular the administration of noble gases under hyperbaric conditions, to provide neuroprotection, in particular neuroprotection against neuronal damage resulting from an impact trauma to the head or spinal column.

The satisfactory treatment of traumatic brain injury (TBI) represents a major unmet clinical need. It has been estimated that each year, in the United States alone, approximately 1.5 million people will sustain TBI. At least 15% will be hospitalized and 3% will die. For approximately 80,000 of those that are hospitalized and survive, the injury will herald the onset of long-term disability. A significant number of those that are injured but are not admitted to hospital are also likely to suffer significant health care problems.

One of the difficulties in developing strategies to treat traumatic brain injury is the highly heterogeneous nature of both the initial injury and its subsequent pathological development. Severe life-threatening head trauma will inevitably involve mechanisms of developing injury which differ from those that occur following a mild contusion. Nonetheless, a number of common neuronal and biochemical mechanisms are thought to be involved. It seems to be generally agreed that, while the primary injury will cause immediate mechanical damage to both the vasculature and to neuronal tissue, there follows a complex series of interacting injury cascades driven by, among other things, ischemia, cerebral edema and axotomy. The fact that these processes lead to a developing "secondary" injury has given some hope that interventions can be devised which arrest the development of injury or minimize its impact.

Xenon is a noble (and thus generally chemically inert) gas whose anesthetic properties have been known for over 50 years. Since the discovery that xenon is an effective antagonist of NMDA receptors, there has been growing interest in its potential use as a neuroprotectant. Xenon has been shown to reduce neuronal injury in a variety of *in vitro* and *in vivo* models, and has a number of attractive features, including the fact that it can be rapidly introduced into the brain and cannot be metabolized. It has been shown to be effective in models that involve hypoxia and/or ischemia.

WO-A-01/08692 discloses the use of xenon as a neuroprotectant, inhibitor of synaptic plasticity, and NMDA receptor antagonist. It is indicated that NMDA receptor activation is a result of hypoxia and ischaemia following head trauma, stroke and cardiac arrest, and that NMDA receptor antagonists are neuroprotective under many clinically relevant circumstances, including ischemia, brain trauma, neuropathic states, and certain types of convulsions.

WO-A-03/092707 discloses the use of xenon for the control of neurological deficits associated with cardiopulmonary bypass.

WO-A-06/018655 discloses the use of xenon in the preparation of a medicament for treating, preventing and/or alleviating one or more anesthetic induced neurological deficits.

According to a first aspect, the present invention provides the use of a noble gas for the manufacture of a medicament for administration under hyperbaric conditions to provide neuroprotection.

It has surprisingly been found that administering xenon under moderate hyperbaric conditions provides significantly increased neuroprotective effects. Moreover, it has been found that administration of other noble gases, such as helium in particular, under hyperbaric conditions also has a significant neuroprotective effect. So far as the inventors are aware, neither the neuroprotective effect of hyperbaric conditions, nor the ability of noble gases other than xenon to provide neuroprotection under such conditions, has previously been suggested or predicted.

The noble gases consist of those elements found under Group 18 of the periodic table, i.e. the currently known noble gases are helium, neon, argon, krypton, xenon and radon.

As used herein, the term "neuroprotection" means protecting a neural entity, such as a neuron, at a site of injury, for example an ischemic or traumatic injury. The term "administration under hyperbaric conditions" means administration to the patient whilst exposed to a hyperbaric environment, such as when the patient is within a hyperbaric chamber. The terms "hyperbaric" and "normobaric" have their ordinary meaning in the art, i.e. normobaric means a pressure equal to about 1 atm (normal air pressure at sea level; approximately 0.1 MPa) and hyperbaric means a pressure above normobaric pressure.

In a preferred embodiment, the neuroprotection is against neuronal damage resulting from an impact trauma.

In a preferred embodiment, the hyperbaric conditions constitute a pressure of no more than about 3 atm (0.3 MPa). More preferably the hyperbaric conditions constitute a pressure of between about 1.5 atm (0.15 MPa) and about 2.8 atm (0.28 MPa), still more preferably about 2.0 atm (0.20 MPa) to about 2.5 atm (0.25 MPa), and most preferably about 2.2 atm (0.22 MPa) to about 2.3 atm (0.23 MPa).

The medicament is preferably a gaseous medicament for administration by inhalation or simulated inhalation. Alternatively, where the noble gas is xenon this may be administered parenterally by injection or transdermally as known in the art. As used herein, the term "simulated inhalation" refers to those situations where a patient is or may be unable to breath unassisted, and is therefore placed on to a heart-lung machine (also known as a pump oxygenator or cardiopulmonary bypass machine) or similar device. In such circumstances, the gaseous medicament is administered to the oxygenator of the heart-lung machine, which simulates the function of the patient's lungs in allowing oxygen (and the noble gas) to diffuse into (and carbon dioxide to diffuse out of) blood drawn from the patient. The oxygen enriched blood is then pumped back to the patient.

In a preferred embodiment, the noble gas is xenon, helium, or a mixture of xenon and helium. Where the noble gas comprises xenon, the partial pressure of xenon in the administered medicament is preferably no more than about 0.8 atm (0.08 MPa). Preferably the partial pressure of xenon in the administered medicament is about 0.1 atm (0.01 MPa) to about 0.7 atm (0.07 MPa), more preferably about 0.2 atm (0.02 MPa) to about 0.6 atm (0.06 MPa), most preferably about 0.4 atm (0.04 MPa). Where the noble gas comprises helium, the partial pressure helium in the administered medicament is preferably such as is needed to bring the total pressure of the administered medicament to pressures equal to the preferred hyperbaric conditions, as discussed above.

In one embodiment, the noble gas is admixed with air so as to provide an administered medicament having an air partial pressure of about 1 atm (0.1 MPa). This is achieved by adding the noble gas to normobaric air so as to provide a hyperbaric mixture. In those embodiments where the noble gas is xenon only, it is preferred that the medicament is administered at a pressure of between about 1.2 atm (0.12 MPa) and about 2 atm (0.2 MPa), more preferably about 1.4 (0.14 MPa) to about 1.8 atm (0.18 MPa). While this may result in a xenon partial pressure slightly above the preferred partial pressures discussed above, the inventors have found that this is compensated by the beneficial effects of the overall hyperbaric conditions. The overall hyperbaric conditions are, in this instance, preferably slightly below the generally most preferred hyperbaric conditions, again as discussed above, because the inventors have also found that if too high levels of xenon are used then, surprisingly, xenon may exhibit neurotoxic effects. In another embodiment, the noble gas is admixed with a gas or gas mixture comprising oxygen so as to provide an administered medicament having a nitrogen partial pressure equal to or less than about 0.8 atm (0.08 MPa). Preferably the administered medicament has a nitrogen partial pressure of less than about 0.4 atm (0.04 MPa), and most preferably the gas mixture is essentially free of nitrogen. The inventors found that nitrogen appears to exacerbate neuronal injury, and it is therefore preferred that the presence of this gas is minimised.

Preferably, the oxygen partial pressure in the administered medicament is about 0.2 atm (about 0.02 MPa, i.e. the same as in normobaric air).

According to a second aspect, the present invention provides a method of providing neuroprotection comprising placing a patient in need of neuroprotection in a hyperbaric environment.

As indicated above, it has surprisingly been discovered that hyperbaric conditions *per se* have a neuroprotective effect. Moreover, it has been discovered that this neuroprotective effect is sufficiently strong that, within certain ranges of pressure, even if the hyperbaric conditions are achieved without adding a noble gas and instead by adding a nitrogen containing gas (which, as noted above, has been found to exacerbate neuronal damage) the hyperbaric conditions will be sufficient to provide an enhanced neuroprotective effect in spite of increased exposure to nitrogen.

Thus, in one embodiment the hyperbaric environment may consist of hyperbaric air or a hyperbaric mixture of air and added nitrogen. Where the hyperbaric environment consists of a mixture of normobaric air and added nitrogen, which is being inhaled by the patient, it is preferred that the pressure of the hyperbaric environment is no more than about 2.8 atm (0.28 MPa).

In a much preferred embodiment, however, the method further comprises administering a noble gas to the patient while the patient is in the hyperbaric environment.

Other preferred features of the second aspect of the present invention, such as but not limited to the hyperbaric conditions, conditions to be treated, and composition and administered pressure of the gas or gas mixture comprising the noble gas, are as discussed above in relation to the first aspect of the present invention.

According to a third aspect, the present invention provides an apparatus comprising: a hyperbaric chamber suitable for housing a human or animal patient; a container holding xenon; and means for delivering the xenon to a patient inside the chamber.

The apparatus is, in particular, suited for carrying the method of the second aspect of the invention, where the method comprises administering a noble gas comprising xenon.

In one embodiment, the xenon delivery means comprise a face mask or mouthpiece in flow communication with an outlet to the container, so as to allow inhalation of xenon by the patient.

In another embodiment, the xenon delivery means comprise a conduit allowing xenon from the container to admix with the atmosphere inside the chamber.

In a further embodiment, the xenon delivery means comprise a heart-lung machine. Operation of such a machine has been briefly described above.

The invention is further described below by way of nonlimiting example, with reference to the accompanying figures in which:
Figure 1 depicts, both in overview (A) and in a close-up view of part thereof (B), an experimental apparatus used to induce reproducible focal injury in organotypic brain slices;
Figure 2 is a graph showing the distribution of fluorescent intensity for two propidium iodide (PI) stained hippocampal slices, one with and one without focal trauma, 72 hours after trauma - also shown (inserts) are fluorescent images of a hippocampal slice without trauma (upper left) and with trauma (lower left), and a graph (upper right) of propidium iodide staining of cells permeabilized with 70% ethanol;
Figures 3A and B are bar charts showing (A) the development of injury including the site of focal injury ("Total injury") and (B) the development of injury excluding the site of focal injury ("Secondary injury");
Figure 4 is a graph showing the effects of added pressures of helium and nitrogen on the development of total injury; and
Figure 5 is a graph showing the effects of added pressures of xenon on the development of total injury.

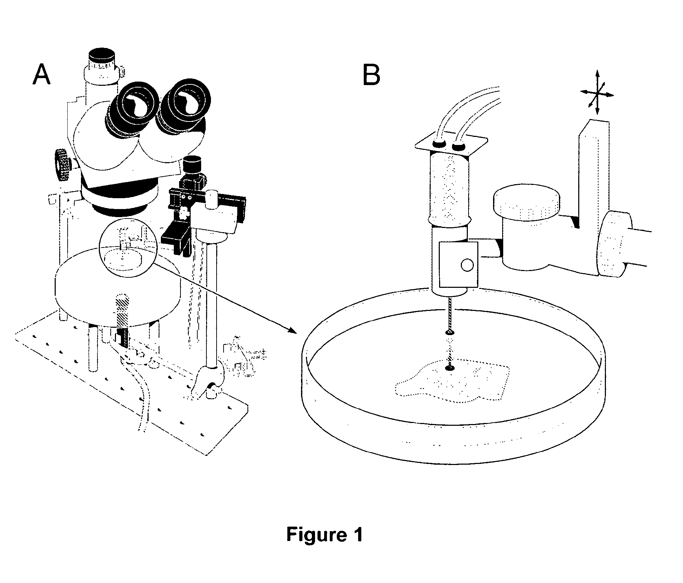

The properties of xenon and other gases were investigated in an *in vitro* model of traumatic brain injury (as described below in greater detail, under the heading "Example"). The model chosen involved creating a focal mechanical trauma centered on the CA1 region of cultured hippocampal brain slices, and assessing neurological injury using propidium iodide staining. The apparatus used to induce focal injury in the organotypic brain slices is shown in Figure 1. A small solenoid retains a stylus 5 mm above a cultured hippocampal slice which is positioned using a micromanipulator. A fiber-optic light source illuminates the slice from beneath. As shown in (B) the stylus is constrained in a glass capillary and positioned just above the CA1 region of the hippocampus.

Hippocampal brain slices after two weeks in culture, so called organotypic slices, maintain heterogeneous populations of cells whose synaptic contacts reflect, at least to some extent, the *in vivo* state. They represent a useful compromise between models that use dissociated cells cultures and those that use intact animals. The nature of the focal trauma, and the subsequent slowly developing secondary injury, bears a sufficiently close relationship to the *in vivo* situation to provide a useful model in which to test possible treatments. A limitation of the model is that it excludes any injury pathways that are as a consequence of ischemia and/or hypoxia, or are as a consequence of changes in systemic parameters (e.g. blood pressure) and focuses primarily on the mechanical component of injury. Xenon, however, has already been shown to be effective in models which involve hypoxia and ischemia, whereas it was not known whether xenon would show any particular efficacy in the present model of brain trauma.

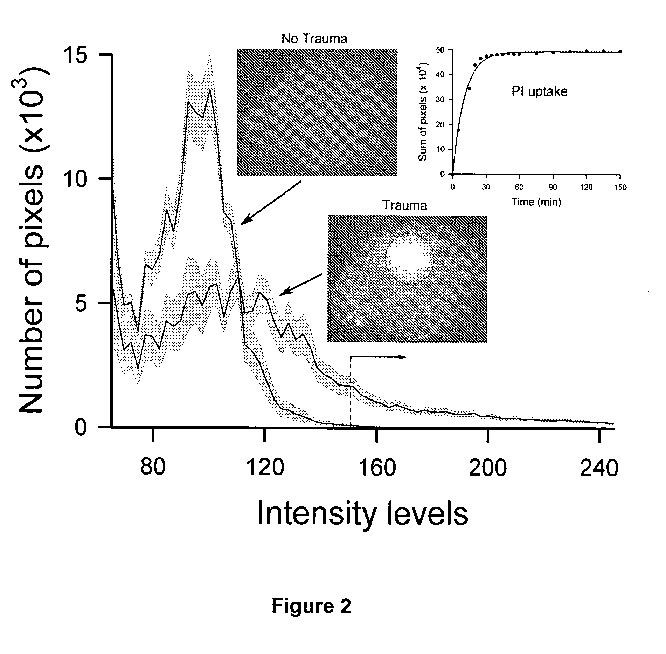

Figure 2 shows the distribution of fluorescent intensity for two propidium iodide (PI) stained hippocampal slices. One slice was subject to a focal trauma and one what not, the images being taken 72 hours after trauma. PI is a membrane-impermeable dye that can only enter cells with damaged cell membranes, that becomes highly fluorescent on binding to DNA, and that can therefore be used to quantify cell injury in terms of fluorescence. As is illustrated in Figure 2, trauma caused a marked rightwards shift in the intensity distribution.

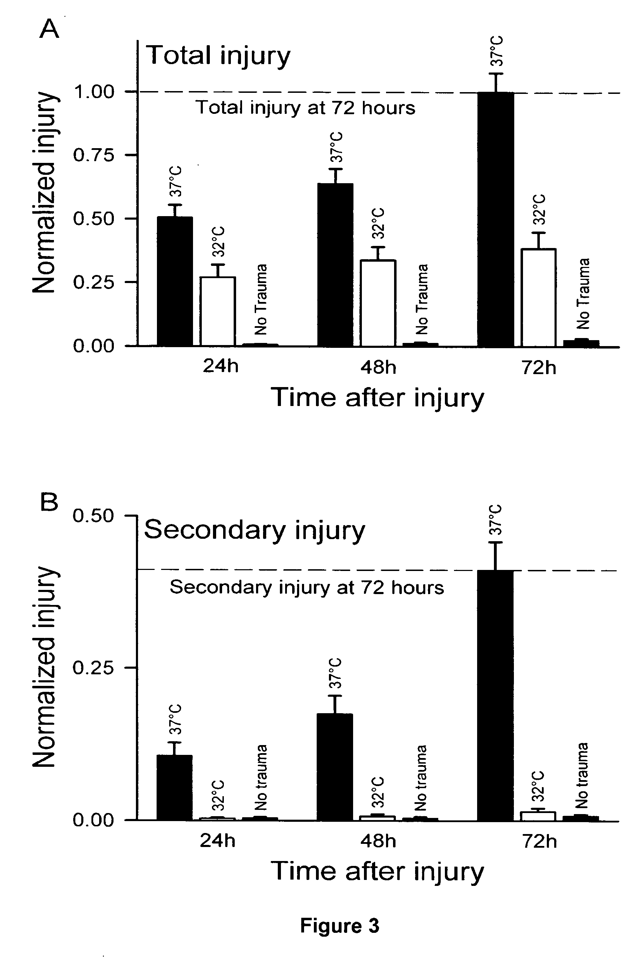

Figures 3A and B chart the development of injury in the hippocampal slices over time. Figure 3A shows the development of injury including the site of focal injury ("Total injury") and Figure 3B shows the development of injury excluding the site of focal injury ("Secondary injury"). The solid bars represent the data obtained for slices maintained at 37°C while the open bars represent the data obtained for slices maintained at 32°C. The bars labeled "no trauma" represent data from brain slices which have not suffered traumatic injury. The dashed line in Figure 3A represents the total injury under control conditions after 72h (at 37°C), which has been normalized to unity (as described infra in greater detail). The error bars are SEMs and the data are from an average of 12 slices.

Within hours of injury being induced bright punctate propidium iodide staining was evident, a pattern of staining that has been shown to correlate well with neuronal damage assessed by morphological changes. The injury slowly increased with time and approximately doubled between 24h and 72h (Figure 3A). When the region of the slice centered at the focal point of injury was excluded from the analysis, the development of injury was even more dramatic. This secondary injury distant from the site of mechanical trauma, which starts from baseline levels, progressively increased (Figure 3B).

The difference between this secondary injury and the total injury which included the region close to the focal point of trauma was particularly evident when the effects of subjecting the brain slices to temperatures equivalent to moderate hypothermia were investigated. When the brain slices were incubated at 32°C following trauma, the secondary injury was almost completely eliminated (Figure 3B). In contrast, this level of hypothermia reduced the total injury by a more limited extent (Figure 3A), presumably because the initial mechanical injury that was caused by the focal trauma was irreversible.

The neuroprotective effects of hypothermia are well known and have been shown in a variety of laboratory models of injury. Indeed, a similar observation has been made before using an equivalent model to the one employed herein, although the finding that hypothermia is much more effective against secondary rather than primary injury goes a step further than the findings in the earlier study.

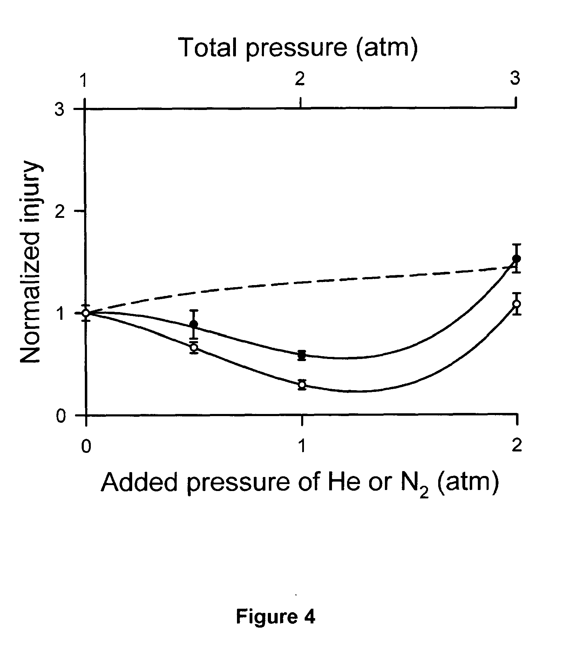

Having established a test protocol that produced a consistent and reproducible injury, the effects of varying other factors were investigated. Most surprisingly, it was found that subjecting the brain slices to added pressures of helium (adding helium to normobaric air to provide a hyperbaric environment) provided potent neuroprotection (Figure 4). Figure 4 shows the effects of added pressures (pressure added to 1 atm (0.01 MPa) of air) of helium and nitrogen on the development of total injury. The effects of helium are shown as open circular symbols and those of nitrogen are shown as filled circular symbols. The solid lines are drawn by eye. Error bars are SEMs for an average of 14 slices.

The effects of added helium are considered to be the effects of added pressure *per se* for a number of reasons. It is widely accepted that even at high pressures, helium is unlikely to exert any direct pharmacologic effects, due to its very low solubility in biological tissues. Indeed, when the effects of helium have been directly compared to those of hydrostatic pressure, it has almost invariably been concluded that any observed effects are due to pressure *per se.* The excitable effects of high pressures (the so-called high pressure nervous syndrome) are well known among divers, but these effects generally occur at pressures slightly above those used in the present investigations. Nonetheless, significant effects on neuronal excitability have been described using *in vitro* systems over the pressure ranges used herein. While the effects of pressure are complex, one consistent finding is that excitatory synapses are depressed, most likely because of suppression of neurotransmitter release. Reductions in the levels of glutamate could be neuroprotective because they would tend to mitigate against excitotoxicity and, without being bound by any theory, this may be a basis for the neuroprotection observed at up to 2 atmospheres (0.2 MPa) of added pressure.

Given that helium at these low pressures is unlikely to be exerting any effects on its own, the significantly worse injury outcome that occurred when helium was replaced by nitrogen (Figure 4) is probably due to some deleterious effect of nitrogen *per se.* The dashed line in Figure 4 is constructed by subtracting the effects of helium (considered to be the effects of pressure *per se*) from the effects of nitrogen to give the theoretical effect of increasing nitrogen levels *per se* (i.e. excluding the concurrent effects of increased pressure).

As nitrogen has a considerably higher fat solubility than helium (implying a higher partitioning into brain tissue), and as the effects of nitrogen narcosis are evident at only a few atmospheres, it is perhaps not surprising that even low pressures of nitrogen exert some pharmacologic effects, although it could not have been predicted whether these would have been beneficial or harmful. If nitrogen is indeed deleterious, then its replacement by helium should be neuroprotective even at normobaric pressures, and by an extent inverse to the deleterious effects that can be predicted from the dashed line in Figure 4 of a normobaric partial pressure of nitrogen. When this experiment was performed the observed a level of injury (after 72 hours) in a brain slice exposed to normobaric air with helium substituted for nitrogen (0.67 ± 0.10) was indeed very close to that predicted theoretically (0.75).

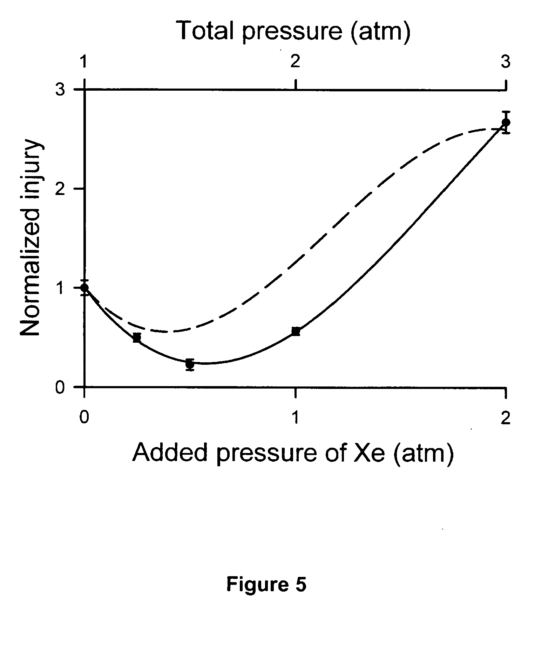

The effects of added pressures of xenon on the development of total injury were also investigated, the results being shown in Figure 5. The dashed line in Figure 5 is constructed by subtracting the effects, as depicted in Figure 4, of helium (assumed to be those of pressure *per se*) from the effects of xenon to give the theoretical effect of increased xenon levels *per se* (i.e. excluding the concurrent effects of increased pressure). Error bars are SEMs for an average of 13 slices. As the data in Figure 5 shows, xenon exhibited marked neuroprotection at low pressures, but then showed toxicity at the highest pressure used. Both of these features, neuroprotection and toxicity, are due to xenon itself as shown by dashed line in Figure 5. Xenon toxicity has not been previously reported, although to the inventors' knowledge such high levels of xenon have not previously been tested.

Further details of the above investigations are provided below.

### EXAMPLE

### Materials and Methods

Unless otherwise stated, all chemicals were obtained from Sigma Chemical Company Ltd. (Poole, Dorset, UK.)

### Hippocampal organotypic slices

Organotypic hippocampal slice cultures were prepared as reported by Stoppini L, et al in "A simple method for organotypic cultures of nervous tissue" J Neurosci Methods 1991;37:173-82, subject to some modifications. Briefly, brains were removed from seven-day-old C57/BL6 mice pups (Harlan UK Ltd., Bicester, Oxfordshire, UK) and placed in ice-cold "preparation" medium. The preparation medium contained Gey's balanced salt solution and 5 mg ml⁻¹ D-glucose (BDH Chemicals Ltd., Poole, Dorset, UK). The hippocampi were removed from the brains and 400µm thick transverse slices were prepared using a McIllwain tissue chopper. Slices were transferred into ice-cold preparation medium, gently separated and then placed onto tissue culture inserts (Millicell-CM, Millipore Corporation, Billerica, MA) which were inserted into a six-well tissue culture plate. The wells contained "growth" medium which consisted of 50% Minimal Essential Media Eagle, 25% Hank's balanced salt solution, 25% inactivated horse serum, 2 mM L-glutamine, 5 mg ml⁻¹ D-glucose (BDH) and 1% antibiotic-antimycotic suspension. Slices were incubated at 37°C in a 95% air/5^{%} CO₂ humidified atmosphere. The growth medium was changed every three days. Experiments were carried out after 14 days in culture.

### Traumatic injury and hyperbaric gas chamber

After the hippocampal slices had been in culture for 14 days, the growth medium was changed to "experimental" medium. The experimental medium was serum-free and consisted of 75% Minimal Essential Media Eagle, 25% Hank's balanced salt solution, 2 mM L-glutamine, 5 mg ml⁻¹ D-glucose, 1% antibiotic-antimycotic suspension and 4.5 µM propidium iodide.

The trauma to the slices was produced with a specially designed apparatus (Figure 1) which was based on published descriptions (Adamchik Y, et al "Methods to induce primary and secondary traumatic damage in organotypic hippocampal slice cultures" Brain Res Brain Res Protoc 2000;5:153-8 and Adembri C, et al "Erythropoietin attenuates post-traumatic injury in organotypic hippocampal slices" J Neurotrauma 2004;21:1103-1220,21). Under a stereomicroscope a stylus was positioned 5 mm above the CA1 region of the hippocampus using a three-axis micromanipulator. The stylus dropped onto the slice with an impact of 3.5 µJ when power to a small electromagnet was switched off. This energy was selected so that the stylus dropped onto the tissue and did not rebound; this produced a consistent and reproducible focal traumatic injury. The distal part of the stylus was smooth and rounded in order to prevent perforation of the slice and the impact produced a focal injury with a diameter of 750 ± 17 µm (mean ± SD).

After traumatizing the CA1 region, the culture trays were transferred to a small pressure chamber which contained a high-speed fan for rapid gas mixing. The pressure chamber was capable of maintaining a constant pressure of up to six atmospheres for several days. The pressure chamber was housed in an incubator which was set at 37°C for normothermic experiments or 32°C for experiments at moderate hypothermia. Subsequently, the pressure chamber (gas volume 0.925 litre) was flushed with humidified control gas (95% air and 5% CO₂) for 5 min at 5 litres min⁻¹ which ensures better than 99.99% gas replacement. After flushing, the pressure chamber was sealed, and slices under these conditions were considered to be the "injury controls" (75% nitrogen/20% oxygen/5% CO₂).

For the experiments under hyperbaric conditions, the chamber was pressurized with experimental gas (xenon, helium or nitrogen between 0.25 atm (0.025 MPa) and 2 atm (0.2 MPa), added *in addition* to the 1 atmosphere (0.1 MPa) of 95% air/5% CO₂ and then sealed. To test the effects of helium and xenon under normobaric conditions, the pressure chamber was flushed with humidified gas mixtures containing either 75% helium/20% oxygen/5% CO₂ or 75% xenon/20% oxygen/5% CO₂ for five minutes and then sealed.

After 24h in the chamber, the slices were imaged using a fluorescent microscope (as described in greater detail below). After completing the imaging, the slices were transferred back to the pressure chamber and the appropriate gas mixture and pressure re-established. This procedure was repeated at 48h and 72h post trauma. It should be noted that, for all gas mixtures and for all pressures, the partial pressures of oxygen and carbon dioxide were fixed at 0.2 atm (0.02 MPa) and 0.05 atm (0.005 MPa), respectively.

### Quantifying cell injury

Propidium iodide (PI) is a membrane-impermeable dye that only enters cells with damaged cell membranes. Inside the cells it binds principally to DNA and becomes highly fluorescent, with a peak emission spectrum in the red region of the visible spectrum. An epi-illumination microscope (Nikon Eclipse 80, Kingston upon Thames, Surrey, UK), and a low-power (2x) objective were used to visualize the PI fluorescence. A digital video camera and software (Micropublisher 3.3 RTV camera and QCapture Pro software, Burnaby, British Columbia, Canada) were used to capture the images. The images were analyzed using the ImageJ software (http://rsb.info.nih.gov). Red, green and blue channels were recorded, but only the red channel was used and the distribution of intensities was plotted as a histogram with 256 intensity levels.

Slices under standard control conditions (incubated in the chamber for 72h at 37°C with 95% air and 5% CO₂) showed a well-defined peak in the intensity distribution (Figure 2) which fell rapidly to zero. In contrast, following trauma, the peak in the intensity distribution was lower, broader and shifted to higher intensity levels (Figure 2).

As a measure of trauma, the number of pixels above an intensity threshold of 150 were integrated (indicated by the arrow and dashed line in Figure 2), which under the experimental conditions used provided a robust quantitative measurement of PI fluorescence, and hence of cell injury. Injury could then be expressed relative to the total injury observed after 72h under control conditions (75% nitrogen, 20 oxygen, and 5% CO₂; 1 atm (0.1 MPa); and 37°C), which was normalized to unity. Thus, for example, if under a particular set of test conditions the integrated number of pixels above an intensity threshold of 150 (calculated as above) is half that present under control conditions, the injury produced by those test conditions is characterized as 0.5 the normalized injury.

Two different measures of injury were used: "total" injury, which was defined as the increase in fluorescence over the entire slice; and "secondary" injury, which was the increase in fluorescence over the slice but excluding the region covering the focal injury. The region covering the focal injury was excluded by masking the area of focal injury in the image prior to integration. The mask was a circle with a diameter of 1000 mm (outline depicted by the dotted circle in the lower image in Figure 2), which was sufficiently large to cover the region of focal injury.

Because the light output from the mercury lamp changed over time, the exposure time was adjusted to take this into account. This was done by recording fluorescence from a glass slide standard (Fluor-Ref, Omega Optical, Brattleboro, VT) and adjusting the exposure time accordingly.

### Results

Within three hours of producing the focal injury, increased fluorescence at the site of injury was evident, indicating almost immediate cell injury in this region. The fluorescence intensity continued to slowly increase, however, both within the region of focal injury as well as over regions of the brain slice distant from the site of injury ("secondary" injury). The slowly developing increase in PI fluorescence was due to increasing cell injury rather than increasing PI binding to cells that have already died. This can be deduced from the data in the inset to Figure 2 which shows that when cells are permeabilized using ethanol, PI binding is very rapid (with a half-time of about 7 minutes), and that equilibration is complete after approximately 30 minutes. In contrast, cell injury following trauma progresses slowly and continues to increase for at least 72h post injury, the longest time point investigated.

The filled bars in Figure 3A show the increase in total injury at 37°C with time, normalized to the injury observed at 72h, while the filled bars in Figure 3B show injury for the same slices but where the focal injury has been excluded from the analysis. The damage in the absence of traumatic injury (labeled "no trauma") was negligible at all time points. A comparison of the data in Figures 3A and B show that the secondary injury constitutes an increasing proportion of the total injury as time progresses.

Moderate hypothermia (32°C) greatly reduced the development of injury after 24h. The open bars in Figure 3A show that the development of total injury with time is very modest at 32 °C and therefore, proportionately, the protection due to hypothermia became greater with increasing time. For example, at 24h, hypothermia reduced total injury by about 46- while at 72h, hypothermia reduced injury by 62%. Figure 3B shows the effects of hypothermia on secondary injury. When the focal site of injury is excluded, it can be seen that the effects of hypothermia in reducing injury are even more pronounced. At 72h, for example, injury is reduced by over 96%.

Having established a protocol that produced a consistent and reproducible injury, the effects of the noble gas helium on the development of injury were investigated. The results are presented in Figure 4, where the open circular symbols show that for pressures up to about 1 atm (0.1 MPa) of added helium, injury decreased, but as higher pressures of helium were added, the injury worsened and came back close to control levels at an added pressure of about 2 atm (0.2 MPa; about 3 atm (0.3 MPa) total pressure). The experiments were then repeated, except with nitrogen substituted for helium. Qualitatively the effects of nitrogen were very similar (filled circular symbols in Figure 4) to those observed with helium, although at all pressures the outcome was significantly worse.

Because helium is most unlikely to be exerting a pharmacologic effect at these low pressures it is reasonable to conclude that the effects observed with helium are due to pressure *per se.* On this basis, the theoretical effect of increased nitrogen levels (independent of the effects of pressure) was calculated (dashed line in Figure 4) by subtracting the effects of helium (constituting the effects of added pressure *per se*) from the effects of added pressures of nitrogen. Nitrogen can thus be seen to be detrimental in a fashion that is roughly linear with increasing amounts. It follows that if helium replaced nitrogen in air under normobaric conditions then this would be predicted to be neuroprotective also, if only because it was replacing the deleterious effects of the nitrogen normally present in the air. This prediction was tested by using a mixture of 75% helium/20% oxygen/5% CO₂, from which it was found that the observed degree of normalized injury after 72 hours (0.67 ± 0.10) was indeed close to that predicted simply by the absence of nitrogen (0.75).

Next, the effects of xenon, which has as noted above been shown to have neuroprotective properties in a variety of *in vitro* and *in vivo* models of neuronal injury, were investigated. The results are presented in Figure 5, and show that low added pressures of xenon provided marked neuroprotection but that this was reversed at the highest pressure tested where significant neurotoxicity was observed.

Following the logic outlined above, theoretical effect of increased xenon levels (independent of the effects of pressure) can also be calculated by subtracting the effects of helium (concluded to be the effect of pressure *per se*). The result of this calculation is shown as the dashed line in Figure 5. The validity of this analysis can also be tested by measuring the neuroprotection afforded by 75% xenon under normobaric conditions. The protection that would be predicted would be that expected of this level of xenon (calculated from the dashed line in Figure 5), plus the benefits of removing the deleterious effect of nitrogen at the same level (calculated from the dashed line in Figure 4) as in this case xenon would be replacing nitrogen. The predicted level of injury after 72 hours is calculated to be 0.59 and the observed degree of injury was found to be 0.50 ± 0.04, a reasonable agreement between predicted and experimental values.

Whilst the invention has been described with reference to various specific embodiments, it will be appreciated that variations and modification made be made without departing from the spirit and scope of the invention.

## Claims

1. Use of a noble gas for the manufacture of a medicament for administration under hyperbaric conditions to provide neuroprotection.

2. A use as claimed in claim 1, wherein the neuroprotection is against neuronal damage resulting from an impact trauma.

3. A use as claimed in claim 1 or 2, wherein the hyperbaric conditions constitute a pressure of no more than 0.3 MPa.

4. A use as claimed in any preceding claim, wherein the medicament is a gaseous medicament for administration by inhalation or simulated inhalation.

5. A use as claimed in claim 4, wherein the noble gas is xenon, helium, or a mixture of xenon and helium.

6. A use as claimed in claim 5, wherein the noble gas is xenon or a mixture of xenon and helium, and the partial pressure of xenon in the administered medicament is no more than 0.08 MPa.

7. A use as claimed in any one of claims 4 to 6, wherein the noble gas is admixed with air so as to provide an administered medicament having an air partial pressure of about 0.1 MPa.

8. A use as claimed in any one of claims 4 to 6, wherein the noble gas is admixed with a gas or gas mixture comprising oxygen so as to provide an administered medicament having a nitrogen partial pressure equal to or less than 0.08 MPa.

9. A use as claimed in claim 8, wherein the gas mixture is essentially free of nitrogen.

10. A use as claimed in claim 8 or 9, wherein the oxygen partial pressure in the administered medicament is about 0.02 MPa.

11. A noble gas for use in providing neuroprotection by administration under hyperbaric conditions.

12. The noble gas of claim 11, wherein the neuroprotection is against neuronal damage resulting from an impact trauma.

13. The noble gas of claim 11 or 12, wherein the hyperbaric conditions constitute a pressure of no more than 0.3 MPa.

14. The noble gas of any one of claims 11 to 13, wherein the noble gas is for administration by inhalation or simulated inhalation.

15. The noble gas of claim 14, wherein the noble gas is as further defined in any one of claims 5 to 10.

## Patentansprüche

1. Verwendung eines Edelgases für die Herstellung eines Medikamentes zur Verabreichung unter Überdruckbedingungen, so daß für einen Nervenschutz gesorgt wird.

2. Verwendung nach Anspruch 1, wobei der Nervenschutz auf eine Nervenschädigung zielt, die durch ein Schlagtrauma entsteht.

3. Verwendung nach Anspruch 1 oder 2, wobei die Überdruckbedingungen einen Druck von nicht mehr als 0,3 MPa darstellen.

4. Verwendung nach einem der vorstehenden Ansprüche, wobei das Medikament ein gasförmiges Medikament für die Verabreichung durch Inhalation oder simulierte Inhalation ist.

5. Verwendung nach Anspruch 4, wobei das Edelgas Xenon, Helium oder ein Gemisch von Xenon und Helium ist.

6. Verwendung nach Anspruch 5, wobei das Edelgas Xenon oder ein Gemisch von Xenon und Helium ist und der Partialdruck von Xenon im verabreichten Medikament nicht mehr als 0,08 MPa beträgt.

7. Verwendung nach einem der Ansprüche 4 bis 6, wobei das Edelgas mit Luft gemischt ist, so daß ein verabreichtes Medikament mit einem Partialdruck der Luft von etwa 0,1 MPa bereitgestellt wird.

8. Verwendung nach einem der Ansprüche 4 bis 6, wobei das Edelgas mit einem Gas oder Gasgemisch, das Sauerstoff umfaßt, gemischt ist, so daß ein verabreichtes Medikament mit einem Partialdruck von Stickstoff von 0,08 MPa oder weniger bereitgestellt wird.

9. Verwendung nach Anspruch 8, wobei das Gasgemisch im wesentlichen frei von Stickstoff ist.

10. Verwendung nach Anspruch 8 oder 9, wobei der Partialdruck von Sauerstoff im verabreichten Medikament etwa 0,02 MPa beträgt.

11. Edelgas für die Verwendung bei der Bereitstellung eines Nervenschutzes durch Verabreichung unter Überdruckbedingungen.

12. Edelgas nach Anspruch 11, wobei der Nervenschutz auf eine Nervenschädigung zielt, die durch ein Schlagtrauma entsteht.

13. Edelgas nach Anspruch 11 oder 12, wobei die Überdruckbedingungen einen Druck von nicht mehr als 0,3 MPa darstellen.

14. Edelgas nach einem der Ansprüche 11 bis 13, wobei das Edelgas für die Verabreichung durch Inhalation oder simulierte Inhalation ist.

15. Edelgas nach Anspruch 14, wobei das Edelgas wie in einem der Ansprüche 5 bis 10 näher angegeben ist.

## Revendications

1. Utilisation d'un gaz noble pour la fabrication d'un médicament à administrer dans des conditions hyperbares pour fournir une neuroprotection.

2. Utilisation selon la revendication 1, dans laquelle la neuroprotection s'applique contre un dommage neuronal résultant d'un traumatisme par impact.

3. Utilisation selon la revendication 1 ou 2, dans laquelle les conditions hyperbares constituent une pression d'au plus 0,3 MPa.

4. Utilisation selon l'une quelconque des revendications précédentes, dans laquelle le médicament est un médicament gazeux à administrer par inhalation ou inhalation simulée.

5. Utilisation selon la revendication 4, dans laquelle le gaz noble est le xénon, l'hélium, ou un mélange de xénon et d'hélium.

6. Utilisation selon la revendication 5, dans laquelle le gaz noble est le xénon, ou un mélange de xénon et d'hélium, et la pression partielle du xénon dans le médicament administré ne dépasse pas 0,08 MPa.

7. Utilisation selon l'une quelconque des revendications 4 à 6, dans laquelle le gaz noble est mélangé avec de l'air, de façon à fournir un médicament administré ayant une pression partielle d'air d'environ 0,1 MPa.

8. Utilisation selon l'une quelconque des revendications 4 à 6, dans laquelle le gaz noble est mélangé avec un gaz ou un mélange gazeux comprenant de l'oxygène, de façon à fournir un médicament administré ayant une pression partielle d'azote égale ou inférieure à 0,08 MPa.

9. Utilisation selon la revendication 8, dans laquelle le mélange gazeux est essentiellement exempt d'azote.

10. Utilisation selon les revendications 8 ou 9, dans laquelle la pression partielle d'oxygène dans le médicament administré est d'environ 0,02 MPa.

11. Gaz noble à utiliser dans la fourniture d'une neuroprotection par administration dans des conditions hyperbares.

12. Gaz noble selon la revendication 11, dans lequel la neuroprotection s'effectue contre les dommages neuronaux résultant d'un traumatisme par impact.

13. Gaz noble selon la revendication 11 ou 12, dans lequel les conditions hyperbares constituent une pression d'au plus 0,3 MPa.

14. Gaz noble selon l'une quelconque des revendications 11 à 13, dans lequel le gaz noble est destiné à une administration par inhalation ou inhalation simulée.

15. Gaz noble selon la revendication 14, dans lequel le gaz noble est comme mieux défini dans l'une quelconque des revendications 5 à 10.
